# EUROPEAN PATENT SPECIFICATION

(11) **EP 3 247 292 B1**
(45) Date of publication and mention of the grant of the patent: **08.05.2019**
(21) Application number: 16701862.1
(22) Date of filing: 21.01.2016
(51) Int. Cl.: A61B 17/42, A61B 17/44

(54) **OBSTETRIC DEVICE**
GEBURTSHILFEVORRICHTUNG
DISPOSITIF OBSTÉTRIQUE

(30) Priority: 23.01.2015 GB 201501120
(43) Date of publication of application: 29.11.2017
(73) Proprietor: Guy's And St Thomas' NHS Foundation Trust, Great Maze Pond, London SE1 9RT (GB)
(72) Inventor: TYDEMAN, Graham, Glenrothes, Fife KY6 3HY (GB)
(74) Representative: Williams Powell
(86) International application number: PCT/GB2016/050132
(87) International publication number: WO 2016/116758

(56) References cited:
- EP-A1- 1 671 599
- WO-A1-89/06112
- WO-A1-2006/027731
- WO-A1-2008/067204
- FR-A- 936 977
- FR-E- 65 209
- GB-A- 2 302 024
- US-A- 2 194 989
- US-A- 5 224 947
- US-A- 5 810 840
- US-A- 5 865 827
- US-A1- 2012 095 476

## Description

This invention relates generally to an obstetric device, and more particularly to an obstetric device for use in birthing processes particularly birthing processes in which the baby is not delivered via the birthing canal.

It is not uncommon during the birthing process for a pregnant woman to reach full dilation of the cervix and begin pushing the fetus down through the birth canal only to have the fetus become lodged in the birth canal. Often a vacuum or forceps may be used to further assist the delivery. This may, however, worsen the situation by causing the fetus to become more firmly impacted or lodged in the birth canal without achieving delivery. After attempts of vaginal delivery are abandoned, the delivering physician must deliver the fetus through caesarean section.

During the course of a caesarean section, the fetus must be positioned such that the physician can reach behind the head of the fetus to deliver the baby out of the uterine cavity through an incision made for purposes of delivery. Presently, the fetal head is often positioned for such delivery by an assisting physician or nurse. Such an assistant must insert his or her hand up through the birth canal and place his or her fingers against the fetal head to position the head such that the delivering physician can reach behind the head and gently deliver the baby out through the incision in the uterine cavity. Often in such cases, a surgical drape is placed over the legs of the patient and the assistant is essentially working blind under the sterile drape.

The assistant who asserts the force to push the fetal head into position for the delivering physician, operative or surgeon does so with his or her fingers, which requires considerable force and is limited by the length of the assistant's arms and/or fingers, as well as by the assistant's physical strength.

Obstetric devices have been proposed which have a head piece for contacting the baby's head and an elongate tube communicating with the device head piece. The device is introduced via the female vagina and birth canal and when contacting the baby's head permits air communication along the tube to a position below the baby's head. Examples of such devices are disclosed in for example US2013/0325027 or WO2011/058289. EP-1,671,599, US-2,194,989, US-2012/0954476, US-5,224,947, WO-2008/067204, WO-2006/027731, FR-939,977, WO-89/06112 and FR-65,209 disclose suction devices for vacuum extraction of a baby by its head.

What is needed in the art is an obstetrical device which provides for a more efficient and safe delivery of a fetus.

An improved solution has now been devised.

In accordance with the present invention there is provided an obstetric device as specified in claim 1.

In the present invention, a plurality of support lugs are provided extending upwardly from the floor of the cavity, the support lugs being spaced from one another.

The peripheral rim has a peripheral wall extending to the floor of the cavity and the support lug or lugs are spaced from the peripheral wall. This enhances the general ability of the peripheral wall/rim to deform with respect to and independently of the support lugs
In certain embodiments it is preferred that three or more (most preferably four) support lugs are distributed about a single or main communication aperture in the floor of the cavity.

The support lugs have respective inclined upper contact surfaces, preferably inclined toward the centre of the head piece. The contact surfaces are to provide a distributed contact network for contacting the baby's head.

In a preferred embodiment the upper most level of the lugs is positioned below the level of the flexible peripheral rim. The rim deforms under pressure allowing the contact surfaces of the lugs to contact and support the baby's head.

In a preferred embodiment the flexible peripheral rim has an outer, lead-in, inclined surface leading upwardly to an apex of the peripheral rim.

It is preferred that the flexible peripheral rim has an inner, inclined surface leading downwardly from an apex of the peripheral rim.

The nature and technical purpose of the inclined surfaces is described in more detail in relation to the exemplary embodiment.

The head piece may beneficially be provided with an integrally formed tube length communicating with the aperture.

It is preferred that the peripheral rim is inclined at an angle to the axis of the tube length.

The device further comprises an elongate tube extending from the head positioned at a distal end of the tube to a proximal end piece, the proximal end piece being secured to the distal end of the tube and provided with a central bore and a positioning mark or formation provided on the exterior surface of the proximal end piece.

Beneficially, the positioning mark or formation comprises a relief formation (such as a ridge or groove).

The proximal end piece preferably includes wider bore portion connecting with a narrower bore portion, the wider bore formation having formations to enhance securing engagement with the outer surface of the tube.

It is preferred that the narrower bore portion has a diameter corresponding substantially to the bore of the tube.

It is preferred that the proximal end piece has a flared or bulbous termination and the positioning mark or formation is provided on the flared or bulbous termination.

There is also disclosed an obstetric device of multi part construction comprising at least:
a) a head piece for contacting a baby's head;
b) a distal end piece having an orientation mark; and
c) a tube interconnecting the head piece and the distal end piece.

It is preferred that the interconnecting tube is received in respective wide bore portions of the head piece and the distal end piece and has an internal bore dimensioned to correspond to narrower internal bore portions of the head piece and the distal end piece.

Beneficially the interconnecting tube is received in respective wide bore portions of the head piece and the distal end piece, the wide bore portions being provided with gripping surface formations for gripping the outer surface of the interconnecting tube.

Preferably the interconnecting tube has a smooth outer surface.

Preferred features of the alternative aspects are of course mutually beneficial.

These and other aspects of the present invention will be apparent from and elucidated with reference to, the embodiment described herein.

An embodiment of the present invention will now be described, by way of example only, and with reference to the accompany drawings, in which:

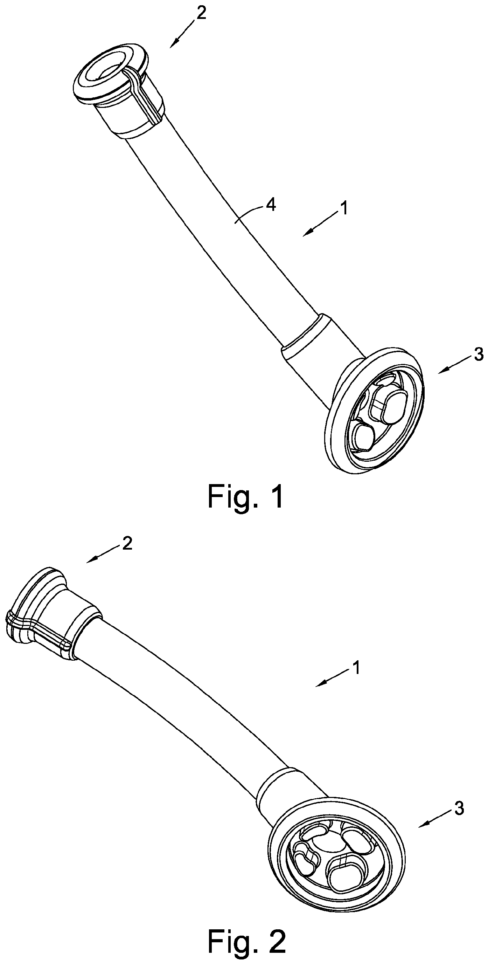

Figures 1 and 2 are different perspective views of an obstetric device in accordance with the invention:
Figures 3A to 3G are various views of the distal head portion of the device of figures 1 and 2, in particular Figure 3D is a view along the section B-B in figure 3E; Figure 3G is a section along A-A in figure 3F; and Figure 3C is a section along B-B in figure 3A;
Figures 4A to 4G are various views of the proximal end portion of the device of figures 1 and 2; in particular, Figure 4E is a section along B-B in figure 4D; and Figure 4G is a section along A-A of Figure 4F;

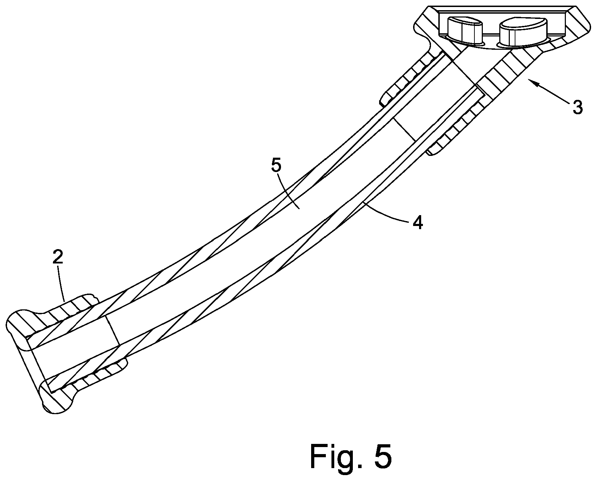

Figure 5 is a longitudinal section of the assembled device.

Referring to the drawings, the obstetric device 1 has certain general features in common with exemplary prior art devices such as those shown in for example US2013/0325027 or WO2011/058289. For example the devices have in common a proximal end portion 2 and a distal head portion 3 connected at either end of an elongate tube 4 having a longitudinally running hollow bore 5.

In accordance with the present invention the proximal end portion 2 and a distal head portion 3 are formed as separate pieces and connected at either end of the elongate tube 4. Each of the distal head piece 3, elongate tube 4, and a proximal end piece 2 are formed of platinum grade silicon 30/40 Shore.

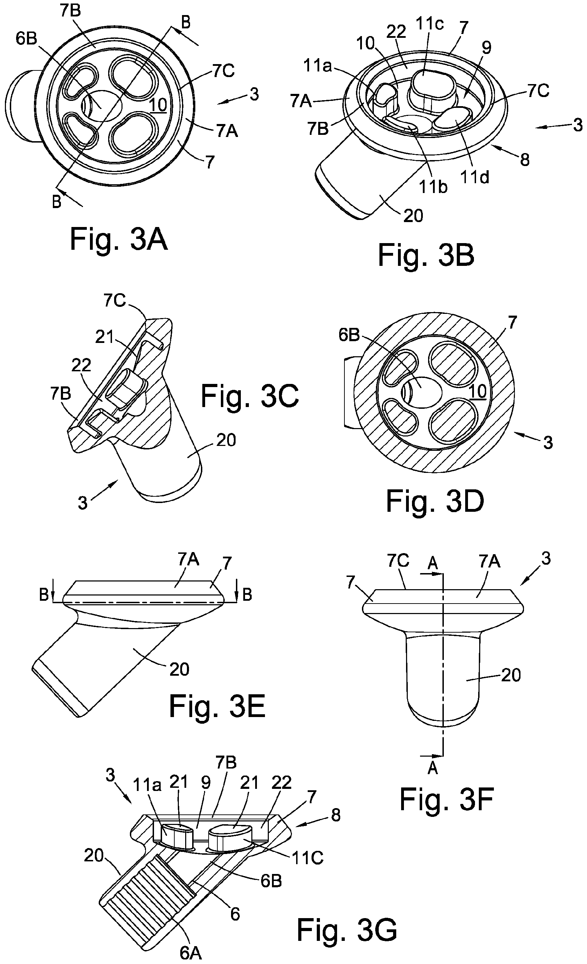

Referring to figures 3A to 3G, the head portion 3 has a stem 20 internally of which is provided a longitudinally running stepped bore 6 communicating with opposed ends having a wide bore portion 6A and a narrower bore portion 6B. Bore portion 6A has a profiled inner surface arranged to provide a grip fit about the external diameter of the interconnecting tube 4 at its end. The profiled inner surface of bore 6A includes a series of spaced arcuate ridge formations providing a secure connection, whilst enabling easy assembly of the device. The narrow bore portion 6B has a bore diameter corresponding to the internal bore 5 of the elongate interconnecting tube 4. At the distal-most portion of the head portion 3 is a contact pad 8 comprising an orbital rim 7 defining a cavity 9 having a floor 10 and upstanding from the floor 10 a plurality of lugs 11a, 11b, 11c, 11d. The lugs 11a, 11b, 11c, 11d have respective upper surface plateaus (see for example 21 in figures 3C and 3G) positioned slightly below the level of the peripheral rim 7. The upper surface plateaus 21 of the lugs 11a, 11b, 11c, 11d are inclined from a an upper edge proximate the peripheral rim 7 to a lower edge toward the centre of the contact pad 8. The bore 6 communicates with the floor 10 of the contact pad 8 via an aperture in the floor 10 and the lugs 11a, 11b, 11c, 11d are distributed about the bore aperture 6. The lugs 11a, 11b, 11c, 11d have upright walls which may be substantially parallel to and spaced from the perimeter wall 22 at the peripheral orbital rim 7. The contact pad 8 is provided at an acute angle (alpha) to the axis of the tube 4. The axis of the tube connector stem 20 of the head is angled with respect to the pad in order to achieve this.

The peripheral orbital rim 7 comprises outer and inner inclined surfaces 7a, 7b meeting at an apex 7c. This arrangement enables the physician's fingers to be guided first upwardly toward the baby's head along lead-in surface 7a and then downwardly along surface 7b under the baby's head when located by the pad 8. The apex 7b is also flexible such that when the baby's head is located it can collapse/deform downwardly such that the surfaces of the lugs 11a, 11b, 11c, 11d contact the baby's head in addition to the peripheral orbital rim 7.

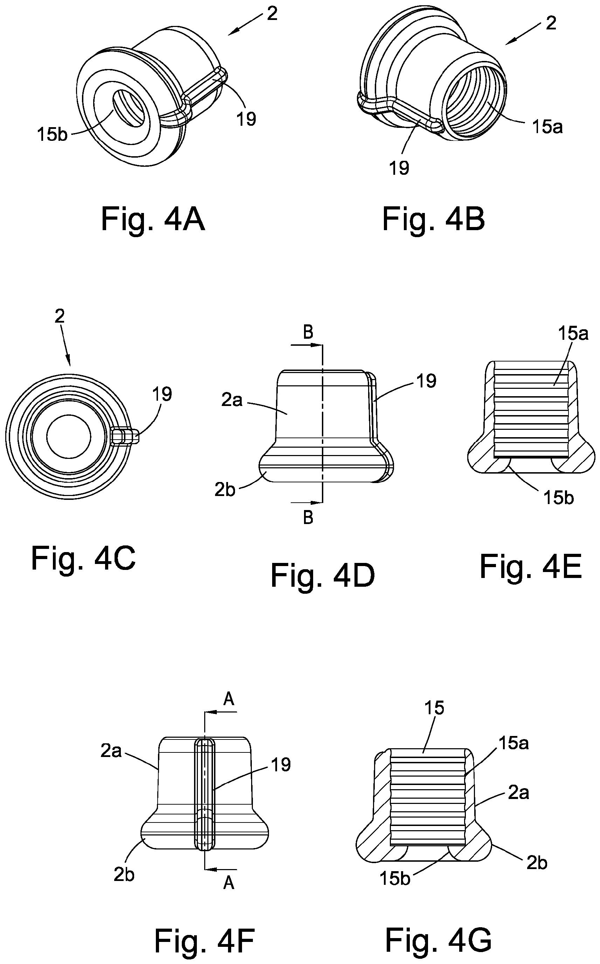

Referring to figures 4A to 4G, the proximal end portion 2 comprises a stepped bore 15 communicating with opposed ends having a wide bore portion 15a and a narrower bore portion 15b. Bore portion 15a has a profiled inner surface arranged to provide a grip or push fit about the external diameter of the interconnecting tube 4 at its end. The profiled inner surface of bore 15a includes a series of spaced arcuate ridge formations providing a secure connection, whilst enabling easy assembly of the device. The narrow bore portion 15b has a bore diameter corresponding to the internal bore 5 of the elongate interconnecting tube 4. The exterior profile of the end portion flares outwardly from a narrower neck 2a to a flared terminal end 2b of wider proportion. The outer surface is also provided with a longitudinally running orientation formation in the form of a ridge formation 19 which importantly extends to lie over the flared terminal end section 2b.

Instead of a longitudinally running ridge formation 19 a groove or other formation could be provided or even a non-profiled mark, in order to function as the orientation formation 19. However a formation having a tactile quality is preferred. The orientation formation 19 can be used to indicate the position of the head. An advantage of the present invention is that the proximal end portion can be fitted to the tube 4 in any angular position or orientation to suit the operative, such that the position of the head 3 is indicated by reference to the position of the orientation formation 19 but not interfering with the grip of the user in manipulating the device. For example, upon assembly, the formation could be positioned at the upper or lower surface or rotated through 90 degrees to the preference of the user. It is however important that once fitted the end portion does not rotate relative to the tube 4 once fitted. By having the orientation formation only on the end of the proximal end portion 2, the interconnecting tube 4 can have a smooth outer surface which is preferred from a comfort of use perspective and cleaning. The end piece 2 can be conveniently removed for cleaning.

In operation the device can be used in a similar fashion to the prior art devices described in for example US2013/0325027 or WO2011/058289. However specific advantages of the present invention lie in the following.
1. The soft silicon material of the device are atraumatic to mother and baby.
2. The 3 part construction of the device allows for ease of cleaning and assembly and permits the orientation formation 19 to be set at a position to the user's preference.
3. The provision of the orientation formation 19 on the proximal end portion only allows for ease of manufacture, improved hygiene and patient comfort as the interconnecting tube can have a smooth outer surface.
4. The provision of a central aperture on the pad 8 of the head portion allows for air to enter deep below the head.
5. The provision of the lugs with the inclined surfaces below the level of the orbital peripheral rim allows for good head support over a large area whilst leaving space for the physician's fingers in the cavity of the device head piece. The lugs also prevent introduction of material into the bore of the tube through the head piece pad 8.
6. The inclined lead-in surface 7a and the down surface 7b combined with the flexibility of the head piece pad 8 allow for ease of introduction of the physician's fingers.

It should be noted that the above-mentioned embodiments illustrate rather than limit the invention, and that those skilled in the art will be capable of designing many alternative embodiments without departing from the scope of the invention as defined by the appended claims. In the claims, any reference signs placed in parentheses shall not be construed as limiting the claims. The word "comprising" and "comprises", and the like, does not exclude the presence of elements or steps other than those listed in any claim or the specification as a whole. In the present specification, "comprises" means "includes or consists of" and "comprising" means "including or consisting of". The singular reference of an element does not exclude the plural reference of such elements and vice-versa. The mere fact that certain measures are recited in mutually different dependent claims does not indicate that a combination of these measures cannot be used to advantage.

## Claims

1. An obstetric device comprising a head piece (3) having:
a contact pad (8) comprising flexible peripheral rim (7), a floor (10) spaced from the flexible peripheral rim defining a cavity and a plurality of parallel support lugs (11a-11d) extending upwardly from the floor of the cavity and spaced from the flexible peripheral rim (7) and wherein the peripheral rim is able to deform with respect to and independently of the support lugs;
an air communication aperture (6B) being provided in the floor of the cavity to allow air to enter into the cavity, the support lugs being disposed around the central air communication aperture (6B);
and a tube connector stem (20) coupled to the contact pad and having a bore (6) in communication with the aperture (6A);
wherein the tube connector stem is disposed at an acute angle to the contact pad ;
a tube connected at one end to the tube connector stem and at another end to a proximal end piece (2),
said obstetric device being **characterized in that** the support lugs have respective inclined upper contact surfaces (21), inclined from an upper edge proximate to the peripheral rim to a lower edge toward the centre of the contact pad (8).

2. A device according to claim 1, wherein three or more support lugs (11a-11d) are distributed about the air communication aperture in the floor of the cavity.

3. A device according to any preceding claim, wherein the uppermost level of the lugs (11a-11d) is positioned below the level of the flexible peripheral rim (7).

4. A device according to any preceding claim, wherein the flexible peripheral rim (7) has a peripheral wall extending to the floor of the cavity and the support lug or lugs are spaced from the peripheral wall.

5. A device according to any preceding claim, wherein the flexible peripheral rim (7) has an outer, lead-in, inclined surface (7a) leading upwardly to an apex of the peripheral rim (7c).

6. A device according to any preceding claim, wherein the flexible peripheral rim (7) has an inner, inclined surface (7b) leading downwardly from an apex (7c) of the peripheral rim.

7. A device according to any preceding claim, wherein the elongate tube (4) has a distal end and a proximal end, the head piece (3) being connected to the distal end of the tube; and the proximal end piece (2) is secured to the proximal end of the tube (4) and provided with a central bore (15); wherein the head piece (3), the tube (4) and the proximal end piece (2) have a longitudinally extending hollow bore (5).

8. A device according to any preceding claim, wherein the proximal end piece (2) includes an orientation mark or formation (19) provided on the exterior surface of the proximal end piece (2).

9. A device according to claim 8, wherein the orientation mark or formation comprises a relief formation (such as a ridge or groove) (19).

10. A device according to claim 7, 8 or 9, wherein the proximal end piece (2) can be fitted to the tube (4) in any orientation.

11. A device according to any one of claims 7 to 10, wherein the proximal end piece includes a wider bore portion (15a) connecting with a narrower bore portion (15b), the wider bore portion having formations to enhance securing engagement with the outer surface of the tube (4).

12. A device according to claim 11, wherein the narrower bore portion (15b) has a diameter corresponding to the bore of the tube (4).

13. A device according to any of claims 7 to 12, wherein the proximal end piece (2) has a flared or bulbous termination (2b) and the orientation mark or formation (19) is provided on the flared or bulbous termination.

## Patentansprüche

1. Geburtshilfevorrichtung mit einem Kopfstück (3) mit:
einem Kontaktpad (8), das einen flexiblen Umfangsrand (7), einen Boden (10), der von dem flexiblen Umfangsrand beabstandet ist und der einen Hohlraum definiert, und eine Vielzahl von parallelen Stützlaschen (11a-11d), die sich vom Boden des Hohlraums nach oben erstrecken und von dem flexiblen Umfangsrand (7) beabstandet sind, und wobei der Umfangsrand in der Lage ist, sich in Bezug auf und unabhängig von den Stützlaschen zu verformen;
einer Luftkommunikationsöffnung (6B), die im Boden des Hohlraums vorgesehen ist, um das Eindringen von Luft in den Hohlraum zu ermöglichen, wobei die Stützlaschen um die zentrale Luftkommunikationsöffnung (6B) herum angeordnet sind;
und einem Rohrverbindungsschaft (20), der mit dem Kontaktpad gekoppelt ist und eine Bohrung (6) in Kommunikation mit der Öffnung (6A) aufweist;
wobei der Rohrverbindungsschaft in einem spitzen Winkel zum Kontaktpad angeordnet ist;
einem Rohr, das an einem Ende mit dem Rohrverbindungsschaft und an einem anderen Ende mit einem proximalen Endstück (2) verbunden ist,
wobei die Geburtshilfevorrichtung **dadurch gekennzeichnet ist, dass** die Stützlaschen jeweils geneigte obere Kontaktflächen (21) aufweisen, die von einer oberen Kante nahe dem Umfangsrand zu einer unteren Kante in Richtung der Mitte des Kontaktpads (8) geneigt sind.

2. Vorrichtung nach Anspruch 1, wobei drei oder mehr Stützlaschen (11a-11d) um die Luftkommunikationsöffnung im Boden des Hohlraums verteilt sind.

3. Vorrichtung nach einem vorhergehenden Anspruch, wobei die oberste Ebene der Laschen (11a-11d) unterhalb der Ebene des flexiblen Umfangsrandes (7) positioniert ist.

4. Vorrichtung nach einem vorhergehenden Anspruch, wobei der flexible Umfangsrand (7) eine Umfangswand aufweist, die sich bis zum Boden des Hohlraums erstreckt, und die Stützlasche oder die Stützlaschen von der Umfangswand beabstandet sind.

5. Vorrichtung nach einem vorhergehenden Anspruch, wobei der flexible Umfangsrand (7) eine äußere, einführende, geneigte Oberfläche (7a) aufweist, die nach oben zu einem Scheitelpunkt des Umfangsrandes (7c) führt.

6. Vorrichtung nach einem vorhergehenden Anspruch, wobei der flexible Umfangsrand (7) eine innere, geneigte Oberfläche (7b) aufweist, die von einem Scheitelpunkt (7c) des Umfangsrandes nach unten führt.

7. Vorrichtung nach einem vorhergehenden Anspruch, wobei das längliche Rohr (4) ein distales Ende und ein proximales Ende aufweist, wobei das Kopfstück (3) mit dem distalen Ende des Rohres verbunden ist; und das proximale Endstück (2) am proximalen Ende des Rohres (4) befestigt und mit einer zentralen Bohrung (15) versehen ist; wobei das Kopfstück (3), das Rohr (4) und das proximale Endstück (2) eine sich in Längsrichtung erstreckende Hohlbohrung (5) aufweisen.

8. Vorrichtung nach einem vorhergehenden Anspruch, wobei das proximale Endstück (2) eine Orientierungsmarke oder Formation (19) umfasst, die auf der äußeren Oberfläche des proximalen Endstücks (2) vorgesehen ist.

9. Vorrichtung nach Anspruch 8, wobei die Ausrichtungsmarkierung oder - formation eine Reliefformation (wie beispielsweise einen Grat oder eine Nut) (19) aufweist.

10. Vorrichtung nach Anspruch 7, 8 oder 9, wobei das proximale Endstück (2) in beliebiger Orientierung an dem Rohr (4) angebracht werden kann.

11. Vorrichtung nach einem der Ansprüche 7 bis 10, wobei das proximale Endstück einen breiteren Bohrungsabschnitt (15a) umfasst, der mit einem engeren Bohrungsabschnitt (15b) verbunden ist, wobei der breitere Bohrungsabschnitt Formationen aufweist, um einen Sicherungseingriff mit der äußeren Oberfläche des Rohres (4) zu verbessern.

12. Vorrichtung nach Anspruch 11, wobei der engere Bohrungsabschnitt (15b) einen Durchmesser aufweist, der der Bohrung des Rohres (4) entspricht.

13. Vorrichtung nach einem der Ansprüche 7 bis 12, wobei das proximale Endstück (2) einen Bördel- oder Wulstabschluss (2b) aufweist und die Orientierungsmarke oder Formation (19) an dem Bördel- oder Wulstabschluss vorgesehen ist.

## Revendications

1. Dispositif obstétrique comprenant une partie de tête (3) ayant :
un tampon de contact (8) comprenant un rebord périphérique souple (7), un plancher (10) espacé du rebord périphérique souple définissant une cavité, et une pluralité d'ergots de support parallèles (11a-11d) s'étendant vers le haut à partir du plancher de la cavité et espacés du rebord périphérique souple (7), et le rebord périphérique étant apte à se déformer par rapport aux des ergots de support et indépendamment de ceux-ci ;
une ouverture de communication d'air (6B) prévue dans le plancher de la cavité pour permettre à de l'air d'entrer dans la cavité, les ergots de support étant disposés autour de l'ouverture de communication d'air (6B) centrale ; et
une tige de raccord de tube (20) couplée au tampon de contact et ayant un alésage (6) en communication avec l'ouverture (6A) ;
la tige de raccord de tube étant disposée selon un angle aigu par rapport au tampon de contact ;
un tube relié au niveau d'une extrémité à la tige de raccord de tube et, au niveau d'une autre extrémité, à une partie d'extrémité proximale (2),
ledit dispositif obstétrique étant **caractérisé par le fait que** les ergots de support ont des surfaces de contact supérieures inclinées respectives (21), inclinées d'un bord supérieur proche du rebord périphérique à un bord inférieur vers le centre du tampon de contact (8).

2. Dispositif selon la revendication 1, dans lequel au moins trois ergots de support (11a-11d) sont répartis autour de l'ouverture de communication d'air dans le plancher de la cavité.

3. Dispositif selon l'une quelconque des revendications précédentes, dans lequel le niveau le plus élevé des ergots (11a-11d) est positionné au-dessous du niveau du rebord périphérique souple (7).

4. Dispositif selon l'une quelconque des revendications précédentes, dans lequel le rebord périphérique souple (7) a une paroi périphérique s'étendant jusqu'au plancher de la cavité et le ou les ergots de support sont espacés de la paroi périphérique.

5. Dispositif selon l'une quelconque des revendications précédentes, dans lequel le rebord périphérique souple (7) a une surface inclinée externe d'introduction (7a) s'étendant vers le haut jusqu'à un sommet du rebord périphérique (7c).

6. Dispositif selon l'une quelconque des revendications précédentes, dans lequel le rebord périphérique souple (7) a une surface inclinée interne (7b) s'étendant vers le bas à partir d'un sommet (7c) du rebord périphérique.

7. Dispositif selon l'une quelconque des revendications précédentes, dans lequel le tube allongé (4) a une extrémité distale et une extrémité proximale, la partie de tête (3) étant reliée à l'extrémité distale du tube ; et la partie d'extrémité proximale (2) est fixée à l'extrémité proximale du tube (4) et comporte un alésage central (15) ; la partie de tête (3), le tube (4) et la partie d'extrémité proximale (2) ayant un alésage creux s'étendant longitudinalement (5).

8. Dispositif selon l'une quelconque des revendications précédentes, dans lequel la partie d'extrémité proximale (2) comprend une formation ou un repère d'orientation (19) prévu sur la surface extérieure de la partie d'extrémité proximale (2).

9. Dispositif selon la revendication 8, dans lequel la formation ou le repère d'orientation comprend une formation en relief (telle qu'une nervure ou une rainure) (19).

10. Dispositif selon la revendication 7, 8 ou 9, dans lequel la partie d'extrémité proximale (2) peut être montée sur le tube (4) dans n'importe quelle orientation.

11. Dispositif selon l'une quelconque des revendications 7 à 10, dans lequel la partie d'extrémité proximale comprend une partie d'alésage plus large (15a) reliée à une partie d'alésage plus étroite (15b), la partie d'alésage plus large ayant des formations pour améliorer l'engagement de fixation avec la surface externe du tube (4).

12. Dispositif selon la revendication 11, dans lequel la partie d'alésage plus étroite (15b) a un diamètre correspondant à l'alésage du tube (4).

13. Dispositif selon l'une quelconque des revendications 7 à 12, dans lequel la partie d'extrémité proximale (2) a une terminaison évasée ou bulbeuse (2b) et la formation ou le repère d'orientation (19) est prévu sur la terminaison évasée ou bulbeuse.
